# FASCICULE DE BREVET EUROPEEN

(11) **EP 0 915 867 B1**
(45) Date de publication et mention de la délivrance du brevet: **06.10.2004**
(21) Numéro de dépôt: 97935617.7
(22) Date de dépôt: 24.07.1997
(51) Int. Cl.: C07D 333/24, C07D 333/20, C07D 409/08

(54) **DERIVES DU THIENYLCYCLOHEXANE POUR LA SYNTHESE DE THIENYLCYCLOHEXYLES**
THIENYLCYCLOHEXAN-DERIVTE ZUR HERSTELLUNG VON THIENYLCYCLOHEXYLEN
THIENYLCYCLOHEXANE DERIVATIVES FOR THIENYLCYCLOHEXYL SYNTHESIS

(30) Priorité: 24.07.1996 FR 9609277
(43) Date de publication de la demande: 19.05.1999
(73) Titulaire: Société de Conseils de Recherches et d'Applications Scientifiques ( S.C.R.A.S.), 75016 Paris (FR)
(72) Inventeur: CAZAUX, Jean-Bernard, F-30390 Aramon (FR); DAFNIET, Michel, F-30400 Villeneuve les Avignon (FR); KAMENKA, Jean-Marc, F-34090 Montpellier (FR); MANGINOT, Eric, F-34000 Montpellier (FR)
(74) Mandataire: Bourgouin, André
(86) Numéro de dépôt international: PCT/FR1997/001382
(87) Numéro de publication internationale: WO 1998/003498

(56) Documents cités:
- FR-A- 2 242 392
- FR-A- 2 271 225
- FR-A- 2 272 660
- FR-A- 2 421 897
- US-A- 4 186 137
- M. MICHAUD ET AL.: "Homochiral structures derived from 1-[1-(2-thienyl)cyclohexyl]piperidine(TCP) are potent non-competitive antagonists of glutamate at NMDA receptor sites" EUROPEAN JOURNAL OF MEDICINAL CHEMISTRYCHIMICA THERAPEUTICA., vol. 29, no. 11, 1994, PARIS FR, pages 869-876, XP002026797
- CHEMICAL ABSTRACTS, vol. 125, no. 5, 29 juillet 1996 Columbus, Ohio, US; abstract no. 52501, OUYANG XIAOHU ET AL.: "Synthesis, radiosynthesis and biological evaluation of fluorinated thienylcyclohexyl piperidine derivatives as potential radiotracers for the NMDA receptor-linked calcium ionophore" XP002026799 & NUCL. MED. BIOL., vol. 23, no. 3, 1996, ENG., pages 315-324,
- M. MOUSSERON ET AL.: "Synthèses et recherche d'activité pharmacologique dans la série de la phencyclidine" CHIMICA THERAPEUTICA, vol. 3, no. 4, 1968, PARIS FR, pages 241-247, XP002026798
- CHEMICAL ABSTRACTS, vol. 85, no. 3, 19 juillet 1976 Columbus, Ohio, US; abstract no. 13673, KALIR A. ET AL.: "1-Phenylcycloalkylamine derivatives" XP002026800 & ISR. J. CHEM., vol. 13, no. 2, 1975, ISRAEL, pages 125-136,

## Description

La présente invention concerne de nouveaux dérivés du thiénylcyclohexane, des procédés pour leur préparation et leur utilisation en tant que produits industriels nouveaux pour la synthèse de dérivés thiénylcyclohexyles, et plus particulièrement de (thiényl-cyclohexyl) amines cycliques telles que décrites dans Eur. J. Med. Chem. (1994) 29, 869-876.

L'invention a pour objet des composés de formule générale I 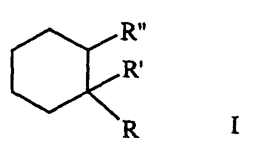 sous forme racémique, de diastéréoisomères ou d'énantiomères dans laquelle
**R** représente le radical cyano ou
-C(O)A dans lequel A représente un radical de formule OR₁ ou NR₂R₃ dans laquelle R₁, R₂ et R₃ représentent, indépendamment, un atome d'hydrogène ou un groupe alkyl éventuellement substitués par aryl
**R'** représente le radical 2-thiényl ou 3-thiényl ;
**R"** représente un radical alkyle éventuellement substitués par aryle ou un radical aryle ainsi que les sels de ces composés.

Le terme alkyle désigne un radical alkyle linéaire ou ramifié comprenant de 1 à 12 atomes de carbone. De préférence, le terme alkyle représente un radical alkyle ayant de 1 à 6 atomes de carbone linéaire ou ramifié et en particulier les radicaux méthyle, éthyle, propyle, isopropyle, butyle, isobutyle, sec-butyle et tert-butyle, pentyle, isopentyle, hexyle, isohexyle.

L'expression aryle représente un radical aromatique, constitué d'un cycle ou de cycles condensés ; chaque cycle peut éventuellement contenir un ou plusieurs hétéroatomes identiques ou différents choisis parmi le soufre, l'azote ou l'oxygène. Des exemples de radical aryle sont les radicaux phényle, naphtyle, thiényle, furyle, pyrrolyle, imidazolyle, pyrazolyle, isothiazolyle, thiazolyle, isoxazolyle, oxazolyle, pyridyle, pyrazinyle, pyrimidyle, benzothiényle, benzofuryle et indolyle.

L'invention a plus particulièrement pour objet les composés de formule générale 1 telle que définie ci-dessus, dans laquelle
**R** représente le radical cyano ;
un radical de formule -C(O)A dans laquelle A représente
un radical de formule OR₁ ou NR₂R₃,
dans laquelle R₁, R₂ et R₃ représentent, indépendamment, un atome d'hydrogène, un radical alkyle
contenant de 1 à 6 atomes de carbone et éventuellement substitués par phényle
**R'** représente le radical 2-thiényl ou 3-thiényl ;
**R"** représente un radical alkyle
contenant de 1 à 6 atomes de carbone et éventuellement substitués par phényle, ou un radical phenyle.

Plus particulièrement, l'invention a pour objet les produits décrits ci-après dans les exemples, en particulier les produits répondants aux formules suivantes :
- le 2-méthyl-1-(2-thiényl) cyclohexane carbonitrile ;
- l'acide 2-méthyl-1-(2-thiényl) cyclohexane carboxylique ;
- le 2-méthyl-1-(2-thiényl) cyclohexane carboxylate d'éthyle ;
- la 2-méthyl-1-(2-thiényl) cyclohexane carboxamide ;
- la N-[α-méthyl-((S)-phénylméthyl)] 2-méthyl-1-(2-thiényl) cyclohexane carboxamide ;
- le 2-méthyl-1-(3-thiényl) cyclohexane carbonitrile ;
- l'acide 2-méthyl-1-(3-thiényl) cyclohexane carboxylique ;
- la 2-méthyl-1-(3-thiényl) cyclohexane carboxamide ;
- le 2-éthyl-1-(2-thiényl) cyclohexane carbonitrile ;
- le 2-propyl-1-(2-thiényl) cyclohexane carbonitrile ;
- le 2-benzyl-1-(2-thiényl) cyclohexane carbonitrile
- le 2-phényl-1-(2-thiényl) cyclohexane carbonitrile
sous forme racémique, de diastéréoisomères ou d'énantiomères.

L'invention a également pour objet un procédé de préparation de composés de formule générale I telle que définie ci-dessus dans laquelle R représente le radical cyano ou un radical de formule -C(O)A dans laquelle A représente un radical de formule OR₁ ou N₂R₃ tel que défini ci-dessus, caractérisé en ce que l'on fait réagir un produit de formule générale 1

R'CH₂R 1

dans laquelle R et R' ont les significations indiquées ci-dessus,
avec un produit de formule générale 2 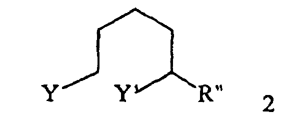 dans laquelle Y et Y' représentent, indépendamment, des groupes partants et R" a la signification indiquée ci-dessus, dans un solvant inerte, en présence d'une base forte, pour donner un produit de formule I.

L'utilisation des composés de départ sous forme racémique conduit à l'obtention des composés de formule I sous forme racémique. L'utilisation d'un composé de formule 2, sous forme (R) ou (S), permet d'obtenir un composé de formule I sous forme d'un énantiomère substantiellement pur.

Dans le produit de formule 2, Y et Y' représentent des groupes partants tel qu'un halogène, et de préférence un atome de chlore, ou un radical aryle ou alkylsulfonate.

Pour la mise en oeuvre du procédé ci-dessus, on utilise un solvant inerte pour la réaction comme, par exemple, l'acétonitrile, le diméthylacétamide ou le diméthylfonmamide, et de préférence le diméthylformamide, en présence d'une base forte. La base forte peut être choisie parmi les produits tels qu'un alkyllithium (comme par exemple un méthyl-, n-butyl- ou t-butyl-lithium) ou un de ses dérivés comme, par exemple, le diisopropylamide lithium (LDA) ou hexaméthyldisilazane lithium (LiHMDS), un alcoolate alcalin (par exemple de méthylate, éthylate, propylate, butylate, tert-butylate, isoamylate ou tert-amylate) ou un hydroxyde de métal alcalin. Le composé de formule 1 est alors ajouté au dit solvant, en présence d'une base forte, à une température comprise entre -80° C et la température ambiante, de préférence entre -20° C et 0° C. Le composé de formule 2 est ensuite ajouté à une température comprise entre -10° C et +10° C, de préférence à une température légèrement en dessous de 0° C. Le milieu réactionnel est ensuite réchauffé à une température comprise entre 20 et 65° C et maintenu sous agitation pendant 15 minutes à quelques heures. La réaction peut être suivie par exemple par chromatographie (couche mince, gazeuse ou liquide selon le cas). La réaction terminée, le solvant est éliminé partiellement sous pression réduite et le résidu traité par un mélange d'eau et de solvant non miscible à l'eau pour extraire le produit de la réaction.

Les produits de formule générale 1 sont commerciaux ou peuvent être obtenus par des méthodes classiques connues de l'homme du métier.

Les produits de formule générale 2 peuvent être obtenus, par exemple, soit par réaction d'un composé organométallique sur la valérolactone, ou un de ses dérivés, suivie d'une réduction puis d'une substitution appropriée des groupements hydroxy, soit par réaction d'un organométallique de formule R"-M-Hal sur un 4-(halogénoformyl), butyrate d'alkyle suivie d'une réduction puis d'une substitution appropriée des groupements hydroxy, soit également par réaction d'un organométallique de formule R"-M-Hal sur le composé approprié de formule générale Y-(CH₂)₄-C(O)Hal suivie d'une réduction puis d'une substitution appropriée du groupement hydroxy, selon le schéma réactionnel 1 ci-dessous. Le composé organométallique tel que défini ci-dessus peut être un composé organomagnésien ou un composé obtenu par échange entre un organomagnésien et un halogénure métallique tel que, par exemple, un chlorure de cuivre, de manganèse ou un autre produit connu de l'homme de l'art.

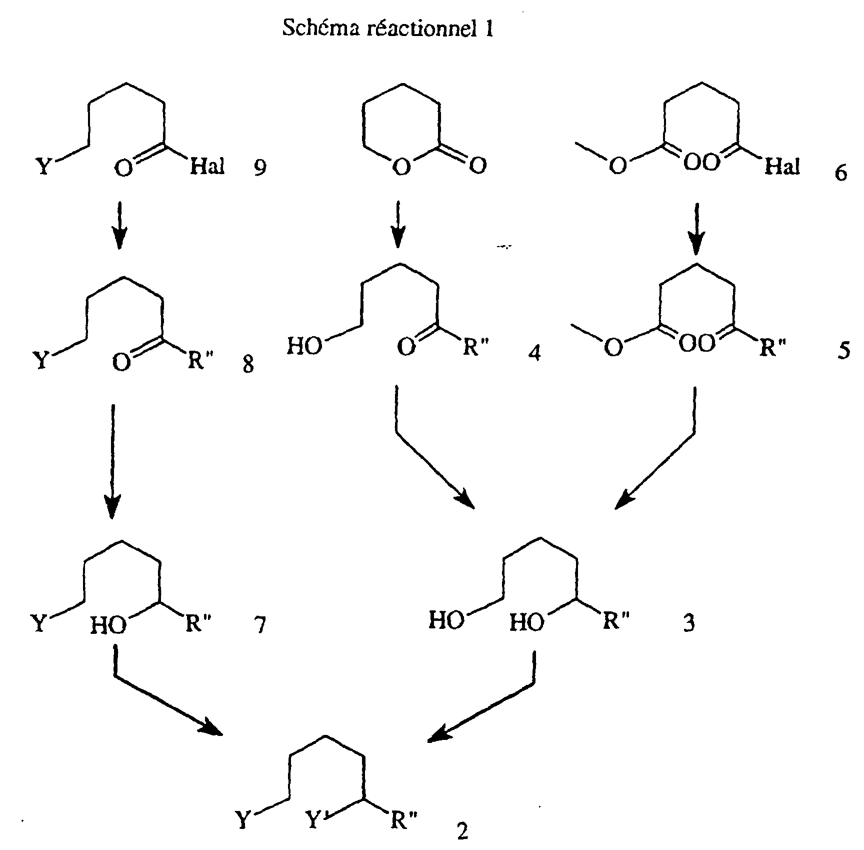

Les composés de formule 2 peuvent également être obtenus selon le schéma réactionnel 2 ci-dessous, par réduction de la cétone de formule R"C(O)(CH₂)₂CH=CH dans laquelle R" a la signification indiquée ci-dessus, puis substitution appropriée du groupe hydroxy ainsi formé, suivie de la transformation de l'alcène en alcool correspondant et enfin de la substitution appropriée du groupe hydroxy ainsi formé.

Les composés de formule 2 sous forme d'énantiomère substantiellement pur, peuvent être préparés selon ce schéma réactionnel 2, par exemple, en incorporant une étape supplémentaire de séparation des alcools de formule R"C(OH)(CH₂)₂CH=CH sous forme d'énantiomère substantiellement pur, puis le traitement de l'un des alcools pour la formation du composé 2 correspondant, sous la forme substantiellement pur souhaitée. Dans le cas où la séparation des alcools sous forme substantiellement pur est difficile, l'alcool sous forme racémique peut être transformé en un autre composé dont les formes énantiomèriques sont plus facilement séparables ; par exemple, on peut faire réagir l'alcool avec l'anhydride phtalique, puis séparer les formes diastéréoisomères du phtalate ainsi formées, et transformer le phtalate, sous forme de diastéréoisomère substantiellement pur, en alcool correspondant, sous forme d'énantiomère substantiellement pur, et enfin traiter cet alcool selon le schéma réactionnel 2.

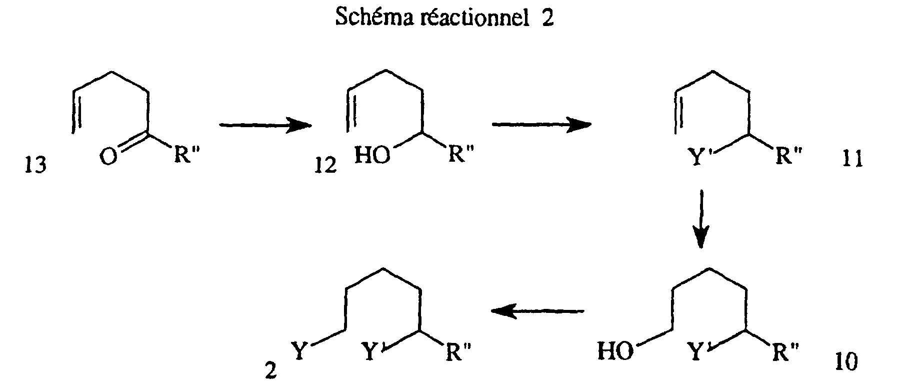

Dans ces schémas réactionnels 1 et 2, Y, Y' et R" ont la signification indiquée ci-dessus et Hal représente un atome d'halogène.

Les composés de formule générale I telle que définie ci-dessus dans laquelle R représente le radical -NR₄R₅, peuvent être obtenus à partir du composé de formule I correspondant dans lequel R représente le radical -C(O)NR₂R₃ dans des conditions opératoires classiques connues de l'homme du métier.

Les composés de formule générale I telle que définie ci-dessus dans laquelle R représente une fonction acide, amide ou ester, peuvent également être obtenus directement ou indirectement à partir du composé de formule I correspondant dans lequel R représente le radical cyano, dans des conditions opératoires classiques connues de l'homme du métier, selon le schéma réactionnel 3 suivant.

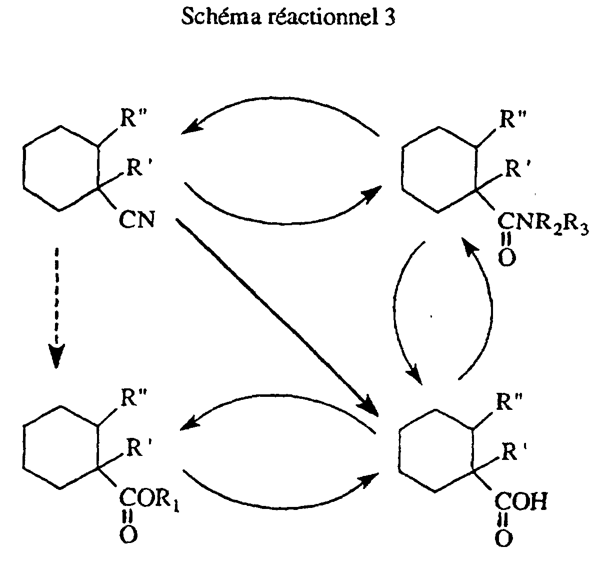

Les composés objet de l'invention peuvent donc être obtenus sous forme racémique à partir des composés de départ sous forme racémique. Ils peuvent également être obtenus avec une conformation cis ou trans prédominante entre les groupements R et R". L'utilisation d'un composé de formule 2, sous forme (R) ou (S), permet d'obtenir un seul énantiomère tout au long de la chaîne synthétique. Par ailleurs, à chaque stade de l'enchaînement, une résolution soit chimique soit enzymatique est possible.

La résolution enzymatique au stade du composé de formule I dans laquelle R représente la fonction nitrile, amide ou ester, est possible avec, par exemple, une nitrilase, une amide hydratase ou acylase, ou une estérase respectivement. Dans le cas de l'ester, on utilise de préférence la Pig Liver Esterase.

La résolution chimique peut être effectuée avec une amine chirale au stade du composé de formule I dans laquelle R représente la fonction acide. L'amine peut être choisie parmi les amines chirales connues de l'homme de l'art. De préférence, on utilise la quinine ou une α-méthyl-benzylamine. Dans le cas de la résolution avec la quinine, le sel de l'acide (+) cristallise préférentiellement. L'obtention de l'acide (-) peut être obtenue par précipitation de son sel avec la D-(+) α-méthyl-henzylamine. De la même façon, la résolution peut être effectuée au stade du composé de formule I dans laquelle R représente une fonction amine par l'emploi d'acides optiquement actifs, et de préférence l'acide tartrique ou un dérivé de l'acide tartrique comme le di-O,O'-toluoyltartrique.

Les composés de formule générale 1 dans laquelle R représente le radical amino, sont des précurseurs des composés de formule II 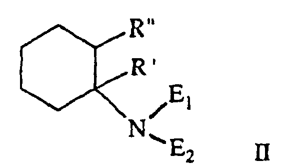 dans lesquels R' et R" ont la significations indiquées ci-dessus et E₁ et E₂ sont liés entre eux et forment avec l'atome d'azote auquel ils sont rattachés, un hétérocycloalkyle ou hétérocycloalkényle. L'expression hétérocycloalkyle ou hétérocycloalkényle désigne un cycloalkyle, saturé ou insaturé, contenant de 3 à 5 atomes de carbones et au moins un hétéroatome. Ce radical peut contenir plusieurs hétéroatomes identiques ou différents. De préférence, les hétéroatomes sont choisis parmi l'oxygène, le soufre ou l'azote. Des exemples de radicaux hétérocycloalkyles ou hétérocycloalkényles sont les radicaux pyrrolidinyle, imidazolidinyle, pyrrazolidinyle, pipéridyle, pipérazinyle, morpholinyle, isothiazolidyle, thiazolidyle, isoxazolidyle, oxazolidyle et 1,2,3,6-tétrahydropyridyle.

Ainsi les composés de formule générale II peuvent être obtenus en faisant réagir sur le composé de formule I correspondant dans lequel R représente le radical amino, un composé de formule Hal-B-Hal dans laquelle Hal représente un atome d'halogène et B une chaîne hydrocarbonée appropriée de 3 à 8 atomes de carbone, saturée ou insaturée, dans des conditions opératoires classiques connues de l'homme du métier. Les composés de formule générale Hal-B-Hal sont commerciaux ou peuvent être obtenus par des méthodes classiques connues de l'homme du métier à partir du diol correspondant de formule HO-B-OH.

L'invention a également pour objet, à titre de produits industriels nouveaux, et notamment à titre de produits industriels nouveaux destinés à la préparation des produits de formule (I), les composés de formule 2 telle définie ci-dessus, sous forme racémique ou d'énantiomère substantiellement pur.

Les exemples suivants sont présentés pour illustrer les procédures ci-dessus .

### Préparation 1 : (+/-) 1,5-hexanediol

Dans un réacteur de 6 l sous atmosphère d'azote, on introduit 560 ml de 4-acétylbutyrate d'éthyle puis 2,8 l de toluène. Dans l'ampoule d'addition, on place 980 ml d'une solution à 15 % en masse de tétrahydrogénoaluminate de lithium dans un mélange toluène / THF, 1 / 2,4. On effectue l'addition du tétrahydrogénoaluminate en 2 heures en laissant la température augmenter progressivement jusqu'au reflux. On maintient au reflux pendant 3 heures. On refroidit alors le réacteur à une température inférieure à 15° C, puis on ajoute très lentement 110 ml d'une solution de soude 5 % et 250 ml d'une autre solution de soude 15 %. On laisse agiter 15 minutes, puis on ajoute 11 de tert-butyl méthyl éther (MTBE). On agite de nouveau 15 minutes, puis on filtre sur 270 g de Clarcel. On rince par 2,8 l de MTBE et on concentre l'ensemble des filtrats. On récupère ainsi 360 g d'une huile assez épaisse correspondant au produit désiré (Rdt = 86 %).
RMN-¹³C (CDCl₃) : 21,7 ; 23,1 ; 32,1 ; 38,5 ; 61,7 ; 67,3.

### Préparation 2 : (+/-) 1,5-dichlorohexane

### Préparation 2 a : à partir du composé 3

Dans un tricol de 31 sous atmosphère d'azote, on introduit 352 g de 1,5-hexanediol puis 11 de toluène. A l'aide d'une ampoule de coulée, on additionne sous bonne agitation en 2 heures, 660 ml de chlorure de thionyle de manière à obtenir un dégagement gazeux régulier et à garder la température inférieure à 40° C. On porte alors progressivement au reflux et on laisse agiter ainsi 1,5 heure. On distille alors l'excès de chlorure de thionyle jusqu'à ce que la température des vapeurs atteigne 109° C. On ajoute alors un volume de toluène égal au volume distillé et on redistille quelques millilitres en contrôlant que la température des vapeurs soit toujours de 109-110° C. On refroidit alors à 20-25° C et on ajoute goutte à goutte 500 ml d'eau. On laisse agiter 15 minutes puis on décante les deux phases. On lave alors la phase organique par 3 fois 400 ml d'une solution aqueuse de bicarbonate de sodium saturée (pH = 7) et par 500 ml d'une solution aqueuse de chlorure de sodium saturée. On élimine le toluène par concentration du milieu, puis on distille sous vide l'huile obtenue. Après élimination d'une tête de 25 g (Eb_{3,75} = 39-54° C), on récupère 292 g d'une huile correspondant au produit désiré (Eb_{3,75} = 55-56° C).
RMN-¹³C (CDCl₃) : 23,9 ; 25,2 ; 31,9 ; 39,4 ; 44,6 ; 58,3.

### Préparation 2 b : à partir du composé 7

En travaillant de la même manière que dans les préparations 12, 13 et 14 ci-dessous et en partant de chlorure de méthyle magnésium, on obtient le composé recherché. Les caractéristiques analytiques sont identiques à celles du produit issu de la préparation 2a.

### Préparation 3 : 5-oxooctanoate de méthyle

Dans un réacteur de 250 ml sous atmosphère d'azote, on introduit 2,82 g de magnésium. On le recouvre de THF et on verse 1 ml de 1-bromopropane. Lorsque la réaction démarre, on additionne goutte à goutte une solution de 9,9 ml de 1-bromopropane dans 150 ml de THF en 1,5 heure. L'addition terminée, on laisse agiter 10 minutes, puis on porte au reflux pendant 3 heures. On refroidit alors le milieu à -80° C, puis on additionne une solution de 17 ml de 4-(chloroformyl)butyrate de méthyle très lentement en 45 minutes. On agite ensuite 1 heure à -70° C, puis on laisse revenir très lentement à 18° C (17 heures). On traite le milieu réactionnel par ajout de 160 ml d'une solution aqueuse saturée de chlorure d'ammonium. On agite 30 minutes, on décante et on réextrait la phase aqueuse par 100 ml d'éther. On concentre les phases organiques. On reprend le résidu dans 200 ml d'éther, puis on lave par deux fois 100 ml d'eau. On sèche sur sulfate de magnésium et on concentre au rotavapor. On obtient ainsi 20,2 g d'une huile correspondant au produit attendu.
RMN-¹³C (CDCl₃) : 13,5 ; 17,1 ; 18,7 ; 32,8 ; 41,2 ; 44,5 ; 51,3 ; 173,4 ; 210,1.

### Préparation 4 : 5-oxoheptanoate de méthyle

En travaillant de la même manière que dans la préparation 3, et en partant de 4,5 ml de bromoéthane, on obtient 9,2 g du produit désiré.
RMN-¹³C (CDCl₃) : 7,6 ; 18,8 ; 32,9 ; 35.7 ; 40,9 ; 51,3 ; 173,4 ; 210,5.

### Préparation 5 : (+/-) 1,5-heptanediol

En travaillant de la même manière que dans la préparation 1, et en partant de 5,4 g de 5-oxoheptanoate de méthyle, on obtient 4,2 g du produit désiré.
RMN-¹³C (CDCl₃) : 9,9 ; 21,7 ; 30,0 ; 32,3 ; 36.2 ; 62,0 ; 72.8.

### Préparation 6 : (+/-) 1,5-octanediol

En travaillant de la même manière que pour la préparation 1, et en partant de 20,0 g de 5-oxooctanoate de méthyle, on obtient 16,9 g du produit désiré.
RMN-¹³C (CDCl₃) : 14,0 ; 18,8 ; 21,7 ; 32,3 ; 36,7 ; 39,5 ; 62,0 ; 71,1.

### Préparation 7 : (+/-) 1,5-dichloroheptane

Dans un réacteur de 100 ml sous atmosphère d'azote, on introduit 20 ml de DMF. On refroidit à 0° C, puis on additionne 5,6 ml de chlorure de thionyle en 10 minutes. On agite ainsi 25 minutes, puis on ajoute une solution de 4,2 g de 1,5-heptanediol dans 8 ml de DMF en 1,5 heure. On agite ainsi 1 heure, puis on laisse revenir à 20-25° C. On chauffe alors à 95° C pendant 45 minutes. On refroidit à 25° C et on ajoute 200 ml d'eau. On extrait par trois fois 80 ml d'éther diéthylique. On lave les phases organiques par deux fois 80 ml d'eau, on sèche sur carbonate de potassium, on filtre et on concentre au rotavapor. On obtient 5,0 g du produit désiré.
RMN-¹³C (CDCl₃) : 10,9 ; 23,9 ; 31,4 ; 32,1 ; 37,2 ; 44,7 ; 65,2.

### Préparation 8 : (+/-) 1,5-dichlorooctane

En travaillant de la même manière que dans la préparation 7, et en partant de 16,5 g de 1,5-octanediol, on obtient 11,8 g du produit désiré.
RMN-¹³C (CDCl₃) : 13,5 ; 19,6 ; 23,8 ; 32,1 ; 37,7 ; 40,5 ; 44,7 ; 63,4.

### Préparation 9 : pent-2-yn-1,5-diol

Dans un tricol de 250 ml, on introduit successivement 26 ml de but-3-yn-1-ol, 55 ml de formaldéhyde 30 %, 0,41 g de carbonate de calcium et enfin 4,9 g d'hydroxyde cuivreux préparé juste avant son utilisation par la méthode traditionnelle. On agite 10 minutes à 20-25° C, puis 96 heures à 80° C. On refroidit à 20-25° C, on filtre la solution obtenue et on concentre. Après distillation sous vide, on récupère 15,3 g du produit recherché (Eb_{0,05} = 107-109° C).
RMN-¹³C (CDCl₃) : 22,8 ; 50,6 ; 60,6 ; 79,9 ; 83,0.

### Préparation 10 : cis pent-2-èn-1,5-diol

Dans un tricol de 250 ml, on dissout 15,1 g de pent-2-yn-1,5-diol dans 150 ml d'acétate d'éthyle. On ajoute alors 0,65 ml de chloroforme puis 0,71 g de palladium sur sulfate de baryum (5 %). On agite 5 minutes, puis on purge le montage à l'hydrogène et on laisse absorber un équivalent d'hydrogène en 6 heures et demie à 20-25° C. On filtre sur célite, on rince par 50 ml d'acétate d'éthyle et on concentre. On récupère ainsi 16,4 g d'une huile correspondant au produit recherché.
RMN-¹H (CDCl₃) : 2,35 (m, 2H) ; 3,61 (t, 2H, CH₂) ; 4,1 (d, 2H, CH₂) ; 4,15 (s, 2H, OH) ; 5,7 (m, 2H, CH=CH).

### Préparation 11 : cis 1,5-dibromo pent-2-ène

Dans un tricol de 100 ml, on introduit 11 ml de tribromophosphine, on refroidit à 0° C puis on additionne goutte à goutte 16,4 g de *cis* pent-2-èn-1,5-diol en trois heures. On laisse revenir lentement à 20-25° C et on agite ainsi 15 heures. On refroidit alors à 0° C, on additionne 30 ml d'eau en 30 minutes. On agite 5 minutes puis on extrait par deux fois 30 ml de dichlorométhane. On lave la phase organique par 15 ml d'eau, on sèche sur sulfate de magnésium, on filtre et on concentre. On récupère ainsi 35 g d'une huile qui correspond au produit attendu avec une pureté suffisante pour être engagé dans des étapes ultérieures.
RMN-¹H (CDCl₃) : 2,55 (m, 2H, CH₂) ; 3,43 (t, 2H, CH₂Br) ; 3,97 (d, 2H, BrCH₂C=C) ; 5,88 (m, 2H, CH=CH).

### Préparation 12 : 6-chloro-1-phényl-hexan-2-one

Dans un tricol de 250 ml, sous atmosphère d'azote, on charge 10 g de chlorure de 5-chlorovaléroyle dans 50 ml de THF anhydre. On refroidit à -20° C et on additionne rapidement 1,06 g de chlorure de cuivre (I). On agite ainsi 40 minutes, puis on additionne en 1,5 heure, 36 ml d'une solution de chlorure de benzylmagnésium 2 M dans le THF. On agite 1 heure à -20° C, puis on laisse remonter à 20° C en 1,5 heure. On refroidit à -10° C et on ajoute 90 ml d'acide chlorhydrique 0,4 M. On agite alors 20 minutes, puis on extrait la phase aqueuse par deux fois 100 ml de MTBE. On lave la phase organique par deux fois 50 ml d'eau, on sèche sur sulfate de magnésium, on filtre et on concentre. On récupère ainsi 13,5 g du produit recherché.

### Préparation 13 : (+/-) 6-chloro-1-phényl-hexan-2-ol

Dans un tricol de 250 ml, on charge 13,1 g de 6-chloro-1-phényl-hexan-2-one dans 75 ml d'éthanol. On refroidit à -5° C et on ajoute en 5 minutes une solution de borohydrure de sodium (2,18 g) dans 15 ml d'eau. On agite 2,5 heures à -5° C, on laisse revenir à 20° C en 1 heure, puis on refroidit à -5° C. On ajoute alors 110 ml d'acide chlorhydrique 0,14 M. On agite 10 minutes, puis on extrait par 3 fois 80 ml de chlorure de méthylène. On sèche sur sulfate de magnésium, on filtre et on concentre. On récupère ainsi 12,3 g du produit attendu.

### Préparation 14 : (+/-) 2,6-dichloro-1-phénylhexane

En travaillant de la même manière que dans la préparation 7 et en partant de 12,3 g de 6-chloro-1-phényl-hexan-2-ol et de 12,9 g de chlorure de thionyle, on récupère 13,3 g du produit attendu.

### Préparation 15 : (+/-) 1,5-dichloro-1-phénylpentane

En travaillant de la même manière que dans les préparations 12, 13 et 14 mais en utilisant du chlorure de phénylmagnésium à la place du chlorure de benzylmagnésium, on obtient le produit recherché.

### Préparation 16 : (+/-) 5-hexen-2-ol

Dans un bicol de 250 ml placé sous argon contenant 2 g de LiAlH₄ et 130 ml d'éther éthylique anhydre, on additionne goutte à goutte à température ambiante 10 g de 5-hexenone diluée dans 20 ml d'éther éthylique anhydre. Le mélange est ensuite porté à reflux pendant 2 heures. On laisse revenir à température ambiante puis on refroidit au bain de glace. 10 ml d'éthanol sont additionnés goutte à goutte puis suivis de 20 ml d'eau distillée. On coupe l'agitation pour laisser déposer le solide blanc formé, puis le surnageant est transféré dans une ampoule à décanter. On sépare les phases : la phase aqueuse est extraite une fois à l'éther (5 ml) et les phases éthérées réunies sont lavées par de l'eau distillée, séchées sur MgSO₄ et concentrées. 9,5 g de produit sont obtenus sous forme d'une huile claire.
RMN-¹H (CDCl₃, δ ppm) : 1,0 (d, CH₃) ; 1,4 (m, CH₂C=C) ; 2,0 (m, CH₂C-O) ; 3,1 (s, OH) ; 3,6 (m, CH-O) ; 4,9 (m, CH₂=C) ; 5,7 (m, CH=C).
RMN-¹³C (CDCl₃, δ ppm) : 23,0 (CH₃) ; 29,9 (CH₂C=C) ; 38,0 (CH₂C-O) ; 67,0 (CH-O) ; 114,3 (CH₂=C) ; 138,3 (m, CH=C).

### Préparation 17 : Δ⁵-2-hexenyl phtalate

On charge dans un monocol de 100 ml, 8 g (80 mmoles) de 5-hexen-2-ol, 12,1 g (80 mmoles) d'anhydride phtalique et 40 ml de pyridine. Le mélange est agité à température ambiante pendant 4 jours. Il est ensuite jeté dans un bécher de 500 ml rempli au 1/3 de glace pilée puis acidifié avec HCl concentré glacé. La solution ainsi obtenue est extraite deux fois au chloroforme glacé. Les phases chloroformiques réunies sont lavées trois fois avec HCl (2N) glacé et trois fois avec une solution saturée de NaCl glacée puis séchées sur MgSO₄ et concentrées. Le concentrât est dissous dans un léger excès de solution de Na₂CO₃ (2N, 40 ml) glacée et cette solution est extraite deux fois à l'éther. Elle est ensuite acidifiée avec HCl (2N) glacée jusqu'à apparition d'un trouble blanc persistant puis extraite au chloroforme. La solution organique est lavée avec une solution saturée de NaCl glacée puis séchée sur MgSO₄ et concentrée. 16 g d'huile claire sont ainsi obtenus. Cette huile, diluée dans 10 ml d'éther de pétrole, est placée en chambre froide (7°C) pendant une nuit avec une agitation douce. Un précipité très blanc de phtalate se forme qui est filtré et séché pour donner 14,3 g de résidu (rendement 72,3 %).

### Préparation 18 : résolution du Δ⁵-2-hexenyl phtalate

Dans un monocol de 250 ml surmonté d'un réfrigérant, un mélange de 14 g de Δ⁵-2-hexenyl phtalate, 18 g de brucine anhydre et 200 ml d'acétone est porté au reflux jusqu'à obtention d'une solution claire. On laisse refroidir à température ambiante. Un sel de brucine précipite qui est filtré, séché et recristallisé une fois dans l'acétone pour donner une masse de 13,6 g. A ce sel dissous à chaud dans 700 ml d'éthanol et 30 ml d'HCl (2N), on ajoute 100 ml d'eau distillée, puis la solution refroidie est extraite quatre fois à l'éther. La phase éthérée, lavée par HCl (2N) et par de l'eau, est séchée sur MgSO₄ et concentrée pour donner 5,5 d'une huile claire. L'huile est hydrolysée avec 50 ml d'une solution de NaOH (2N) dans un monocol de 100 ml et soumise à un entraînement à la vapeur. On obtient 2,2 g d'huile claire caractérisée comme étant le (+)-5-hexen-2-ol. [α]_{D} = +12,2° (c = 2,99 ; Et₂O).

Le (-)-5-hexen-2-ol est obtenu en concentrant la solution du premier filtrat au 1/3 environ de son volume et en la refroidissant au bain de glace. Le sel de brucine ainsi précipité est filtré, séché et traité comme le précédent pour donner 2,5 g de produit. [α]_{D} = -11,1° ([C] = 2,5 ; Et₂O).
RMN-¹H (CDCl₃, δ ppm) : 1,0 (d, CH₃) ; 1,4 (m, CH₂C=C) ; 2,0 (m, CH₂C-O) ; 3,1 (s, OH) ; 3,6 (m, CH-O) ; 4,9 (m, CH₂=C) ; 5,7 (m, CH=C).
RMN-¹³C (CDCl₃, δ ppm) : 23,0 (CH₃) ; 29,9 (CH₂C=C) ; 38,0 (CH₂C-O) ; 67,0 (CH-O) ; 114,3 (CH₂=C) ; 138,3 (m, CH=C).

### Préparation 19 : (+)-5-chloro-1-hexene

Dans un tricol de 100 ml muni d'un réfrigérant, d'un thermomètre et d'une ampoule d'addition sont placés 2,5 g (25 mmoles) de (-)-5-hexen-2-ol et 10 ml de tétrachlorométhane auxquels on additionne goutte à goutte une solution de 8 g de triphénylphosphine dans 10 ml de dichlorométhane. Le mélange est agité une nuit à 25°C puis refroidi à température ambiante et concentré. L'ajout de pentane (20 ml) précipite Φ₃PO qui est filtré et le filtrat est concentré. Le concentré est dilué dans du pentane et placé au bain de glace pendant une heure puis filtré à nouveau. Le filtrat est ensuite passé à travers un gel de silice avec élution au pentane. Après concentration, 1,3 g de produit sont obtenus. L'analyse RMN-¹H et la valeur du pouvoir rotatoire indiquent que le produit obtenu avec inversion de configuration est bien le (+)-5-chloro-1-hexene.
[α]_{D} = +15,5° (c = 2,76 ; Et₂O).
RMN-¹H (CDCl₃, δ ppm) : 1,50 (d, CH₃) ; 1,80 (m, CH₂C=C) ; 2,20 (m, CH₂C-Cl) ; 4,0 (m, CH-Cl) ; 5,1 (m, CH₂=C) ; 5,7 (m, CH=C).

### Préparation 20 : (+)-5-chloro-1-hexanol

Dans un tricol de 50 ml sous argon, on place 1 g de (+)-5-chloro-1-hexene et 10 ml d'éther anhydre puis on refroidit le mélange au bain de glace. 0,5 ml de BH₃:S(CH₃)₂ (10:10,2 M) est additionné goutte à goutte et on agite une heure à température ambiante. Le milieu réactionnel est ensuite oxydé à 0-5°C par 4 ml de NaOH (3N) puis 8 ml de H₂O₂ (30%) et le mélange est agité une heure à température ambiante. Il est ensuite décanté et la phase aqueuse est extraite trois fois par de l'éther. Les phases organiques réunies sont lavées à l'eau distillée, séchées sur MgSO₄ et concentrées pour obtenir 0,7 g (60,8 %) de 5-chloro-1-hexanol. [α]_{D} = +14,2° (c = 2,96 ; Et₂O).
RMN-¹H (CDCl₃, δ ppm) : 1,45 (d, CH₃) ; 1,52 (m, 2CH₂) ; 1,60-1,70 (m, CH₂C=Cl) ; 3,16 (s, OH) ; 3,57 (t, CH₂O) ; 4,1 (m, CH-Cl).

### Préparation 21 : (+)-1,5-dichlorohexane

Les conditions opératoires sont identiques à celles de la synthèse du 5-chloro-1-hexene en utilisant 400 mg (2,93 mmoles) de (+)-5-chloro-1-hexanol, 3 ml de dichlorométhane, 1 g (3,81 mmoles) de triphénylphosphine et 3 ml de tétrachlorométhane. On isole finalement 400 mg (85%) de (+)-1,5-dichlorohexane.
[α]_{D} = +13,56° (c = 2,68 ; Et₂O).
RMN-¹H (CDCl₃, δ ppm) : 1,50 (d, CH₃) ; 1,60-1,85 (m, 6H, CH₂) ; 3,53 (t, 2H, CH₂-Cl) ; 4,1 (m, CH-Cl).

### Exemple 1 : (+/-) cis 2-méthyl-1-(2-thiényl) cyclohexane carbonitrile

Dans un réacteur de 6 l, on charge 430 g de potasse (85 %) dans 1,7 l de DMF. On refroidit alors le milieu à -20° C et on additionne une solution de 191 g de 2-thiénylacétonitrile dans 0,7 l de DMF tout en refroidissant afin de ne pas dépasser une température de -15° C. Cette première phase terminée, on effectue immédiatement l'addition en 1 heure d'une solution de 250 g de 1,5-dichlorohexane dans 0,5 l de DMF en laissant la température s'élever jusqu'à -5° C. Ces deux additions terminées, on retire le bain réfrigérant et on laisse remonter en 40 minutes la température à 20-25° C. On laisse agiter dans ces conditions 4,5 heures, puis on élimine sous vide le DMF. On reprend le milieu obtenu dans 1,5 l d'eau et on extrait par deux fois 500 ml d'heptane. On lave la phase organique par deux fois 500 ml d'eau, on sèche sur sulfate de magnésium, on filtre et on concentre la solution obtenue. On récupère ainsi une huile verdâtre, qui, après distillation sous vide conduit à 267 g d'une huile incolore correspondant au produit désiré (Eb_{2,1} = 90-100° C).
RMN-¹³C (CDCl₃) : 18,0 ; 23,7 ; 25,4 ; 32,0 ; 41,2 ; 42,8 ; 48,1 ; 120,1 ; 124,4 ; 125.1 ; 126,6 ; 145,0.

### Exemple 2 : acide (+/-) cis 2-méthyl-1-(2-thiényl) cyclohexane carboxylique

Dans un réacteur de 4 l, on introduit 266 g de *cis* 2-méthyl-1-(2-thiényl) cyclohexane carbonitrile dans 1 l de diéthylène glycol. Après 5 minutes d'homogénéisation et sous bonne agitation, on ajoute 2,4 kg d'une solution aqueuse de potasse à 60 %. On agite alors à 170° C pendant 24 heures. On refroidit à 20-25° C, puis on dilue par 1 l d'eau. On verse sur 6,5 l d'eau et on lave ce milieu par deux fois 3 l de dichlorométhane. Sous bonne agitation, on acidifie le milieu par ajout de 2,5 1 d'acide chlorhydrique concentré. A température inférieure à 30° C, on extrait ce milieu par deux fois 3 l de dichlorométhane. On réextrait la phase organique par deux fois 2 l de soude (le premier contenant 78 g de soude 99 % et le second 2 g). On écarte la phase organique. On acidifie la phase aqueuse par ajout de 1 l d'acide chlorhydrique (contenant 230 ml d'acide chlorhydrique concentré) et on extrait par deux fois 2 l de dichlorométhane. On lave la phase organique par 2 l d'eau, on traite au noir et au sulfate de magnésium, on filtre et on concentre. On cristallise le solide blanchâtre obtenu dans 1,65 l d'heptane sous agitation. On recueille, après filtration et rinçage par de l'heptane, 178 g d'un solide blanc correspondant au produit souhaité (p.f. = 109° C).
RMN-¹³C (CDCl₃) : 16,1 ; 21,2 ; 22,1 ; 29,0 ; 31,5 ; 37,4 ; 52,5 ; 124,8 ; 125,4 ; 126,5 ; 146,5 ; 180,9.

### Exemple 3 : acide (+) cis 2-méthyl-1-(2-thiényl) cyclohexane carboxylique

On dissout respectivement 30 g d'acide (+/-) *cis* 2-méthyl-1-(2-thiényl) cyclohexane carboxylique et 43,4 g de quinine dans 2,1 l d'acétone à chaud. On mélange ces deux solutions et on laisse alors revenir lentement à 20-25° C, et on agite ainsi 3 heures. On filtre en rinçant par un peu d'acétone. On concentre alors la solution obtenue. On recristallise le sel obtenu dans 860 ml d'acétone. On libère l'acide de son sel isolé par un traitement à l'acide chlorhydrique 10 % et extraction au dichlorométhane. On concentre la phase organique obtenue, après séchage sur sulfate de magnésium et filtration. On recueille ainsi 8,8 g de l'acide (+) recherché (p.f. = 109° C).[α]_{D}²⁰ = +70,95 (éthanol 0,8 %)

RMN-¹³C (CDCl₃): identique au composé de l'exemple 2.

### Exemple 4 : acide (-) cis 2-méthyl-1-(2-thiényl) cyclohexane carboxylique

On concentre à sec le premier filtrat obtenu lors de la préparation de l'exemple 3. On libère l'acide de son sel isolé par un traitement à l'acide chlorhydrique 10 % et extraction au dichlorométhane. On concentre la phase organique obtenue. On dissout respectivement 11,9 g de l'acide libéré et 6,1 g de D (+) α-méthyl-benzylamine dans le MTBE à reflux du solvant. On laisse alors revenir lentement à 20-25° C, et on agite ainsi 3 heures. On filtre en rinçant par un peu de MTBE. On recristallise le sel obtenu dans un mélange MTBE / EtOH 7/3, on filtre et on libère l'acide comme précédemment. On recueille ainsi 4,0 g de l'acide (-) recherché (p.f. : 109° C).[α]_{D}²⁰ = - 72,1 (éthanol 0,8 %).

Cet acide (-) peut également être préparé en faisant réagir le (+)-1,5-dichlorohexane sur la 2-thiénylacétonitrile dans les conditions de mise en oeuvre de l'exemple 1, puis en transformant le nitrile ainsi formé, en acide dans les conditions de mise en oeuvre de l'exemple 2.
RMN-¹³C (CDCl₃) : identique au composé de l'exemple 2.

### Exemple 5 : (+/-) cis 2-méthyl-1-(2-thiényl) cyclohexane carboxylate d'éthyle

Dans un réacteur de 250 ml, on dissout 2,5 g d'acide (+/-) *cis* 2-méthyl-1-(2-thiényl) cyclohexane carboxylique dans 60 ml de toluène. On additionne alors 3 ml de chlorure de thionyle en 15 minutes. On laisse homogénéiser 5 minutes, puis on porte progressivement au reflux en contrôlant le dégagement gazeux (1 heure). On agite au reflux 2 heures. On distille alors l'excès de chlorure de thionyle jusqu'à l'obtention de vapeurs à 108-110° C, puis le toluène. On ajoute 60 ml d'éthanol et on élimine le restant de toluène en distillant l'azéotrope toluène / éthanol jusqu'à l'obtention de vapeurs à 77-78° C. A 20-25° C, on additionne en 20 minutes une solution de 1,5 g d'éthanolate de sodium dans 50 ml d'éthanol. On laisse homogénéiser 5 minutes, puis on porte au reflux pendant 15 heures. On chasse alors l'éthanol, puis on reprend dans 100 ml d'eau, on extrait par trois fois 50 ml d'éther. On lave les phases organiques par 10 ml d'eau, on sèche sur sulfate de magnésium, on filtre et on concentre au rotavapor. On obtient ainsi 2,36 g d'une huile correspondant au produit souhaité.
RMN-¹³C (CDCl₃) : 12,3 ; 13,9 ; 21,8 ; 22,4 ; 29,3 ; 32,1 ; 38,1 ; 52,7 ; 60,5 ; 123,8 ; 124,8 ; 126,2 ; 147,6 ; 174,0.

### Exemple 6 : (+/-) trans 2-méthyl-1-(2-thiényl) cyclohexane carboxylate d'éthyle

En travaillant de la même manière que dans l'exemple 1 mais en partant de 3 g de 2-thiophène acétate d'éthyle à la place du 2-thiénylacétonitrile, on obtient 1,3 g du composé recherché. Il est identifié en comparant les structures du composé obtenu par hydrolyse basique de ce dernier avec les structures du composé de l'exemple 2.

### Exemple 7 : acide (-) cis 2-méthyl-1-(2-thiényl) cyclohexane carboxylique

Dans un tricol de 100 ml, on introduit successivement 50 ml de tampon phosphate (pH = 7), 0,15 ml d'une solution de 1 g de (+/-) *cis* 2-méthyl-1-(2-thiényl) cyclohexane carboxylate d'éthyle dans le propan-2-ol et 0,46 mg de Porcine Liver Esterase Crude. On agite alors fortement l'ensemble 72 heures à 40° C. On refroidit à 20-25° C, on filtre le milieu sur Sephadex puis on alcanise le milieu par ajout de 20 ml de soude 5 %. On extrait par trois fois 100 ml de MTBE. On lave la phase organique par 100 ml d'eau. Les phases aqueuses sont rassemblées puis on acidifie par ajout de 26 ml d'acide chlorhydrique 5 %. On extrait par trois fois 100 ml de MTBE. On lave l'ensemble des phases organiques par 100 ml d'eau, on sèche sur sulfate de magnésium, on filtre et on concentre pour récupérer 0,4 g du composé recherché.
RMN-¹³C (CDCl₃) : identique au composé de l'exemple 4.

### Exemple 8 : acide (+) cis 2-méthyl-1-(2-thiényl) cyclohexane carboxylique

On sèche la première phase MTBE obtenue dans la préparation de l'exemple 7, sur sulfate de magnésium, on filtre et on concentre. On reprend alors l'huile obtenue avec 2 ml d'éthylène glycol. On homogénéise puis on ajoute 4,8 g de potasse aqueuse à 60 %. On agite alors 17 heures à 170° C. On refroidit à 20-25° C, on ajoute 15 ml d'eau. On lave ce milieu par deux fois 10 ml de dichlorométhane. On acidifie par ajout de 5 ml d'acide chlorhydrique concentré. On extrait par deux fois 10 ml de dichlorométhane. On lave l'ensemble des phases organiques par 10 ml d'eau, on sèche sur sulfate de magnésium, on filtre et on concentre pour récupérer 0,45 g du composé recherché.
RMN-¹³C (CDCl₃) : identique au composé de l'exemple 3.

### Exemple 9 : (+/-) cis 2-méthyl-1-(2-thiényl) cyclohexane carboxamide

Dans un réacteur de 2 l sous atmosphère d'azote, on dissout 59 g d'acide *cis* 2-méthyl-1-(2-thiényl) cyclohexane carboxylique dans 600 ml de toluène. On additionne alors 71 ml de chlorure de thionyle en 15 minutes. On laisse homogénéiser 5 minutes, puis on porte progressivement au reflux en contrôlant le dégagement gazeux (4 heures). On distille alors l'excès de chlorure de thionyle jusqu'à l'obtention de vapeurs à 108-110° C. On refroidit dans ces conditions à 10° C, puis on fait barboter de l'ammoniac avec un débit assez important sans refroidir. On laisse la température s'élever jusqu'à 65° C puis redescendre à 20-30° C. L'absorption d'ammoniac étant terminée, on agite 1 heure, puis on dégaze le milieu par bullage d'azote et on ajoute 600 ml d'eau. On décante les deux phases, et on réextrait la phase aqueuse par 300 ml de toluène. On lave l'ensemble des phases organiques par deux fois 250 ml d'eau et on concentre le milieu. Le solide brunâtre obtenu est cristallisé dans 360 ml d'heptane pour conduire à 52 g du produit recherché (p.f. = 100° C).
RMN-¹³C (CDCl₃) : 15,8 ; 20,5 ; 21,9 ; 29,0 ; 30,2 ; 36,2 ; 52,4 ; 124,3 ; 124,8 ; 126,9 ; 148,8 ; 177,1.

### Exemple 10 : (+/-) cis 2-méthyl-1-(2-thiényl) cyclohexylamine

Dans un tricol de 1 l, on dissout 60 g de potasse dans 240 ml d'eau. On refroidit l'ensemble à 0° C. On ajoute d'un coup 7 ml de brome. On laisse homogénéiser 10 minutes puis on additionne rapidement (7 minutes) une solution de 20,5 g de *cis* 2-méthyl-1-(2-thiényl) cyclohexane carboxamide et de 0,5 g d'hydrogénosulfate de tétrabutylammonium dans 220 ml de dichlorométhane. On agite ainsi 1 heure, puis on décante les deux phases. On lave la phase organique par 100 ml d'eau et on concentre le milieu réactionnel afin d'isoler l'isocyanate intermédiaire.

Dans un second tricol de 1 1, on dissout 71 g de soude (99 %) dans 170 ml d'eau. On refroidit l'ensemble à 20-25° C. On additionne rapidement (4 minutes) une solution de 23,2 g de l'isocyanate intermédiaire et de 0,5 g d'hydrogénosulfate de tétrabutylammonium dans 210 ml de MTBE. On agite ainsi 7 heures, puis on décante les deux phases. On extrait la phase aqueuse par 170 ml de MTBE. On lave la phase organique par 70 ml d'eau. On extrait la phase organique par deux fois 200 ml d'eau acidifiée (le premier contenant 100 ml d'acide chlorhydrique 5 % et le second 1 ml). On lave la phase aqueuse par 100 ml de MTBE. On alcalinise par ajout de 170 ml de soude 5 % et on extrait par deux fois 200 ml de MTBE. On lave la phase organique par 100 ml d'eau, on sèche au sulfate de magnésium, on filtre et on concentre. On recueille ainsi 17,2 g d'une huile correspondant à l'amine souhaitée.
RMN-¹³C (CDCl₃) : 15,9 ; 22,1 ; 25,9 ; 30,2 ; 41,7 ; 43,7 ; 56,9 ; 121,1 ; 122,5 ; 126,5 ; 157,4.

### Exemple 11 : (+) cis 2-méthyl-1-(2-thiényl) cyclohexylamine

On dissout 3,0 g de (+/-) *cis* 2-méthyl-1-(2-thiényl) cyclohexanamine et 2,3 g d'acide (-) tartrique dans le minimum d'éthanol 95 % aqueux à chaud. On agite ainsi 12 heures à 20-25° C. On filtre en rinçant par un peu d'éthanol et de l'heptane. On recristallise à quatre reprises les cristaux obtenus dans l'éthanol 95. On reprend le solide dans l'eau et on libère l'amine par ajout de soude 5 %. On extrait alors l'amine à l'éther éthylique, on sèche au sulfate de magnésium et on concentre. On obtient ainsi 0,25 g du produit recherché. [α]_{D}²⁰ = + 14,7 (méthanol, 2 %).
RMN-¹³C (CDCl₃) : identique au composé de l'exemple 10.

### Exemple 12 : (-) cis 2-méthyl-1-(2-thiényl) cyclohexylamine

On concentre les différents filtrats obtenus lors de la préparation de l'exemple 11. On libère l'amine selon le même protocole que précédemment. On la cristallise alors avec l'aide de l'acide (+) tartrique dans le minimum d'éthanol 95 à chaud. On agite ainsi 12 heures à 20-25° C. On filtre en rinçant par un peu d'éthanol et de l'éther de pétrole. On recristallise à trois reprises les cristaux obtenus dans l'éthanol 95. On reprend le solide dans l'eau et on libère l'amine par ajout de soude 5 %. On extrait alors l'amine à l'éther éthylique, on sèche au sulfate de magnésium et on concentre. On obtient ainsi 0,31 g du produit recherché. [α]_{D}²⁰ = - 15,5 (méthanol, 2 %).
RMN-¹³C (CDCl₃): identique au composé de l'exemple 10.

### Exemple 13 : (+/-) cis 2-méthyl-1-(2-thiényl) cyclohexyl pipéridine

Dans un tricol de 250 ml, on dissout 5,7 g de *cis* 2-méthyl-1-(2-thiényl) cyclohexanamine et 5 ml de 1,5-dibromopentane dans 60 ml de sulfolane. Après 5 minutes d'homogénéisation, on ajoute d'un coup 11,7 g de carbonate de potassium et 2,1 g d'iodure de sodium. On laisse homogénéiser de nouveau 5 minutes puis on porte à 80° C pendant 20 heures. On refroidit l'ensemble à 25-30° C. On traite par ajout de 300 ml d'eau et 100 ml de MTBE. On décante les deux phases. On réextrait la phase aqueuse par 150 ml de MTBE. On lave la phase organique par 100 ml d'eau. On extrait la phase organique par deux fois 150 ml d'eau acidifiée (le premier contenant 40 ml d'acide chlorhydrique 5 % et le second 10 ml). On lave les phases aqueuses acides réunies par 100 ml de MTBE et on écarte les phases organiques. On alcalinise les phases aqueuses par ajout de 70 ml de soude 5 % et on extrait par deux fois 100 ml de MTBE. On lave la phase organique d'extraction par 50 ml d'eau, on traite au noir et au sulfate de magnésium, on filtre sur clarcel et on concentre. On recueille ainsi 7,5 g d'un résidu qui est cristallisé dans 150 ml de méthanol. On récupère alors par filtration 5,9 g d'un solide blanc correspondant à la molécule recherchée (p.f. = 81-82° C).
RMN-¹³C (CDCl₃) : 14,0 ; 19,4 ; 23,1 ; 25,1 ; 27,0 ; 29,5 ; 30,1 ; 33,9 ; 46,0 ; 52,7 ; 122,1 ; 124,0 ; 125,9 ; 144,7.

### Exemple 14 : chlorhydrate de (+/-) cis 2-méthyl-1-(2-thiényl)cyclohexyl pipéridinium

Dans un tricol de 250 ml, on introduit 57 ml d'une solution d'acide chlorhydrique 1M dans le MTBE. Sous bonne agitation et sous atmosphère d'azote, on ajoute assez rapidement une solution de 5 g de (+/-) *cis* 2-méthyl-1-(2-thiényl)cyclohexyl pipéridine dans 45 ml de MTBE. L'addition terminée, on laisse agiter 3 heures puis on filtre et on sèche sous 1 torr à 45° C. On recueille ainsi 5,4 g du produit recherché (p.f. = 230-1° C).
RMN-¹³C (CDCl₃) : 15,8 ; 17,8 ; 22,1 ; 22,3 ; 22,5 ; 22,6 ; 26,5 ; 30,2 ; 35,4 ; 46,7 ; 48,9 ; 72,9 ; 127,2 ; 127,6 ; 130,1 ; 136,9.

### Exemple 15 : chlorhydrate de (+) cis 2-méthyl-1-(2-thiényl)cyclohexyl pipéridinium

### 15 a. Résolution chimique

On dissout respectivement 17,53 g de (+/-) *cis* 2-méthyl-1-(2-thiényl)cyclohexyl pipéridine et 12,7 g d'acide (-) di-O,O'-toluoyl tartrique dans le minimum d'isopropanol à chaud. On mélange ces deux solutions et on laisse alors revenir lentement à 20-25° C, et on agite ainsi 4 heures. On filtre en rinçant par un peu d'isopropanol et de l'heptane. On reprend le solide dans l'eau et on libère l'amine par ajout de soude 5 %. On extrait alors l'amine au MTBE, on sèche au sulfate de magnésium et on concentre. On analyse par HPLC sur colonne chirale le taux d'enrichissement en énantiomère (+) et on réitère l'opération de cristallisation trois fois supplémentaires en ajustant la quantité d'acide (-) di-O,O'-toluoyltartrique au rapport stoechiométrique précédent. On recueille de cette manière 3,2 g de l'amine (+) avec un excès énantiomérique supérieur à 99 %. La chlorhydratation de l'amine est effectuée de manière identique à celle décrite lors de la préparation de l'exemple 14, et permet d'obtenir 3,6 g du produit recherché (p.f. = 230-1° C).

La RMN est identique à celle du produit de l'exemple 14.

### 15 b. Synthèse chimique

En pratiquant comme indiqué lors des préparations des exemples 9, 10, 13 et 14 et en utilisant 23,2 g d'acide (+) *cis* 2-méthyl-1-(2-thiényl) cyclohexane carboxylique, on obtient 18,2 g du chlorhydrate (+) recherché (p.f. = 230-1° C). [α]^{D}₂₄ = + 33,0.

La RMN est identique à celle du produit de l'exemple 14.

### Exemple 16 : chlorhydrate de (-) cis 2-méthyl-1-(2-thiényl) cyclohexyl pipéridinium

### 16 a. Résolution chimique

On concentre le premier filtrat obtenu lors de la préparation de l'exemple 15 a., et on effectue l'opération de cristallisation à l'aide de l'acide (+) di-O,O'-toluoyltartrique dans les mêmes conditions que précédemment, trois fois de suite. Après chlorhydratation, on isole 3,7 g du composé recherché (p.f. = 230-1° C).

La RMN est identique à celle du produit de l'exemple 14.

### 16 b. Synthèse chimique

En pratiquant comme indiqué lors des préparations des exemples 9, 10, 13 et 14 et en utilisant 11,5 g d'acide (-) *cis* 2-méthyl-1-(2-thiényl) cyclohexane carboxylique, on obtient 8,1 g du chlorhydrate (-) recherché (p.f. = 230-1° C).

La RMN est identique à celle du produit de l'exemple 14.

[α]^{D}₂₄ = - 32,3

### Exemple 17 : N-[α-méthyl-((S)-phénylméthyl)]cis 2-méthyl-1-(2-thiényl) cyclohexane carboxamide

Dans un réacteur de 100 ml sous atmosphère d'azote, on dissout 2,0 g d'acide *cis* 2-méthyl-1-(2-thiényl) cyclohexane carboxylique dans 20 ml de toluène. On additionne alors 1,3 ml de chlorure de thionyle en 5 minutes. On laisse homogénéiser 5 minutes, puis on porte progressivement au reflux en contrôlant le dégagement gazeux (1 heure). On distille alors l'excès de chlorure de thionyle jusqu'à l'obtention de vapeurs à 108-110° C. On refroidit à 20° C, puis on additionne une solution de 2,55 g d'α-méthyl-(S)-phénylméthylamine dans 40 ml de toluène sans refroidir en 15 minutes. On laisse la température s'élever jusqu'à 40° C puis redescendre à 20-30° C. On agite à 25° C pendant 2 heures, puis on ajoute 40 ml d'eau. On décante les deux phases. On lave la phase organique par deux fois 40 ml d'eau, 40 ml d'acide chlorhydrique et 40 ml d'eau. Après concentration du milieu, le résidu solide obtenu est cristallisé dans 40 ml de pentane pour conduire à 2,4 g du produit recherché.

CCM (SiO₂ 60F254, éluant hexane / éther diéthylique 5/5, révélation UV, Rf = 0,66).

### Exemple 18 : (+/-) cis 2-méthyl-1-(3-thiényl) cyclohexane carbonitrile

Dans un tricol de 500 ml, on met 43 g de potasse dans 160 ml de DMF. On refroidit alors le milieu à -10° C et on ajoute goutte à goutte une solution de 17,6 g de 3-thiénylacétonitrile dans 40 ml de DMF en 30 minutes tout en maintenant la température à -10° C. On agite ainsi 20 minutes. On additionne alors la solution de 24,7 g de 1,5-dichlorohexane dans 40 ml de DMF en 30 minutes à -10° C. On agite ainsi 15 minutes, on laisse revenir à 20-25° C en 1,5 heure. On agite alors pendant 18 heures à 25° C, puis 4 heures à 60° C. On laisse refroidir-à 20-25° C après avoir chassé le DMF sous vide, puis on reprend dans 1 l d'eau. On extrait par 600 ml puis 400 ml d'heptane. On lave la phase organique par 400 ml d'eau puis 400 ml d'une solution de chlorure de sodium. On sèche sur sulfate de magnésium, on filtre et on concentre la solution obtenue. Après distillation sous vide, on obtient 15,2 g d'une huile incolore correspondant au produit désiré (Eb_{0,08} = 114-120° C).
RMN-¹³C (CDCl₃) : 17,9 ; 23,5 ; 25,2 ; 34,7 ; 39,1 ; 40,7 ; 47,8 ; 121,2 ; 121,3 ; 124,7 ; 126,6 ; 142.1.

### Exemple 19 : acide (+/-) cis 2-méthyl-1-(3-thiényl) cyclohexane carboxylique

En partant de 15,0 g de (+/-) *cis* 2-méthyl-1-(3-thiényl) cyclohexane carbonitrile et en procédant de la même manière que celle décrite dans la préparation de l'exemple 2, on obtient après cristallisation dans 4 volumes d'heptane, 11,6 g du composé recherché.

CCM (SiO₂ 60F254, éluant éther diéthylique, révélation UV, Rf = 0,7).

### Exemple 20 : (+/-) cis 2-méthyl-1-(3-thiényl) cyclohexane carboxamide

En partant de 11,6 g de l'acide (+/-) *cis* 2-méthyl-1-(3-thiényl) cyclohexane carboxylique et en procédant de la même manière que celle décrite dans la préparation de l'exemple 9, on obtient après cristallisation dans 5 volumes d'heptane, 12,0 g du composé recherché.
RMN-¹³C (CDCl₃) : 16,0 ; 21,2 ; 22,1 ; 29,1 ; 30,8 ; 35,6 ; 52,1 ; 121,4 ; 126,1 ; 126,9 ; 145.1 ; 177,5.

### Exemple 21 : (+/-) cis 2-méthyl-1-(3-thiényl) cyclohexanamine

En partant de 11,6 g de (+/-) *cis* 2-méthyl-1-(3-thiényl) cyclohexane carboxamide et en procédant de la même manière que celle décrite dans la préparation de l'exemple 10, on obtient 6,9 g d'une huile pratiquement incolore correspondant au composé recherché.
RMN-¹³C (CDCl₃) : 15,2 ; 15,9 ; 21,9 ; 26,1 ; 30,1 ; 40,3 ; 41,8 ; 56,3 ; 118,9 ; 125,2 ; 125,5 ; 152,4.

### Exemple 22 : (+/-) cis 2-méthyl-1-(3-thiényl)cyclohexyl pipéridine

En partant de 5,7 g de (+/-) *cis* 2-méthyl-1-(3-thiényl) cyclohexanamine et en procédant de la même manière que celle décrite dans la préparation de l'exemple 13, on obtient après cristallisation dans 14 volumes de méthanol, 5,9 g du composé recherché.
RMN-¹³C (CDCl₃) : 13,7 ; 19,6 ; 23,0 ; 25,1 ; 27,0 ; 28,8 ; 29,3 ; 32,7 ; 46,0 ; 62,2 ; 120,8 ; 123,1 ; 127,8 ; 141.1.

### Exemple 23 : chlorhydrate de (+/-) cis 2-méthyl-1-(3-thiényl)cyclohexyl pipéridinium

En partant de 0,7 g de (+/-) *cis* 2-méthyl-1-(3-thiényl)cyclohexyl pipéridine et en procédant de la même manière que celle décrite dans la préparation de l'exemple 14, on obtient 0,62 g du chlorhydrate recherché (p.f. = 230° C).
RMN-¹³C (CDCl₃) : 15,5 ; 18,1 ; 22,1 ; 22,3 ; 22,6 ; 25,8 ; 29,9 ; 33,5 ; 46,6 ; 48,8 ; 72,0 ; 126,5 ; 126,6 ; 134,2.

### Exemple 24 : (+/-) [cis 2-méthyl-1-(2-thiényl)cyclohexyl) 1,2,3,6-tétrahydropyridine

En partant de 11.51 g de (+/-) *cis* 2-méthyl-1-(2-thiényl) cyclohexanamine et de 35 g du *cis-*1,5-dibromo pent-2-ène et en procédant de la même manière que celle décrite dans la préparation de l'exemple 13, on récupère 15,2 g d'un solide beige qui est purifié par chromatographie sur 590 g d'alumine (Merck 90) (Eluant heptane). On obtient ainsi 4,81 g du composé recherché.
RMN-¹³C (CDCl₃) : 14,1 ; 19,4 ; 23,0 ; 27,3 ; 29,4 ; 29,9 ; 34,4 ; 42,1 ; 44,5 ; 62,9 ; 122,6 ; 124,5 ; 124,8 ; 126,0 ; 126,5 ; 143,6.

### Exemple 25 : chlorhydrate de (+/-) [cis 2-méthyl-1-(2-thiényl)cyclohexyl] 1,2,3,6-tétrahydropyridinium

En partant de 0,24 g de (+/-) [*cis* 2-méthyl-1-(2-thiényl)cyclohexyl] 1,2,3,6-tétrahydropyridine et en procédant de la même manière que celle décrite dans la préparation de l'exemple 14, on récupère 0,2 g du composé recherché (p.f. = 182-184°C).

### Exemple 26 : (+/-) cis 2-éthyl-1-(2-thiényl) cyclohexane carbonitrile

En travaillant de la même manière que pour la préparation de l'exemple 1 mais en partant de 4,8 g de 1,5-dichloroheptane à la place du 1,5-dichlorohexane, on obtient 2,8 g du produit souhaité.
RMN-¹³C (CDCl₃): 10,1 ; 22,3 ; 23,1 ; 23,9 ; 26,8 ; 41,4 ; 46,9 ; 47,9 ; 118,9 ; 123,0 ; 123,7 ; 125,0 ; 143,8.

### Exemple 27 : (+/-) cis 2-propyl-1-(2-thiényl) cyclohexane carbonitrile

En travaillant de la même manière que dans l'exemple 1 mais en partant de 5,1 g de 1,5-dichlorooctane à la place du 1,5-dichlorohexane, on obtient 3,3 g du produit souhaité.
RMN-¹³C (CDCl₃) : 13,9 ; 23,8 ; 24,9 ; 25,4 ; 28,9 ; 33,9 ; 42,0 ; 47,1 ; 47,8 ; 120,5 ; 124,4 ; 125,2 ; 126,4 ; 145,3.

### Exemple 28 : (+/-) cis 2-benzyl-1-(2-thiényl) cyclohexane carbonitrile

En travaillant de la même manière que dans l'exemple 1 mais en utilisant du 2,6-dichloro-1-phénylhexane à la place du 1,5-dichlorohexane, on obtient le produit recherché.

### Exemple 29 : (+/-) cis 2-phényl-1-(2-thiényl) cyclohexane carbonitrile

En travaillant de la même manière que dans l'exemple 1 mais en utilisant du 1,5-dichloro-1-phénylpentane à la place du 1,5-dichlorohexane, on obtient le produit recherché.

En utilisant le procédé indiqué ci-dessus, on peut également préparer les produits suivants :

**Tableau 1**

| Composé | R | R' | R" |
|---|---|---|---|
| A | CO₂H | 2-thiényl | propyl |
| B | CO₂Et | 2-thiényl | propyl |
| C | C(O)NH₂ | 2-thiényl | propyl |
| D | NH₂ | 2-thiényl | propyl |
| E | CO₂H | 2-thiényl | benzyl |
| F | CO₂Et | 2-thiényl | benzyl |
| G | C(O)NH₂ | 2-thiényl | benzyl |
| H | NH₂ | 2-thiényl | benzyl |
| I | NHMe | 2-thiényl | phényl |
| J | C(O)NHMe | 2-thiényl | 3-chlorophényl |
| K | NH₂ | 2-thiényl | phényl |
| L | CO₂H | 2-thiényl | cyclohexyl |
| M | C≡N | 3-thiényl | butyl |
| N | CO₂H | 3-thiényl | propyl |
| O | C(O)NH₂ | 3-thiényl | propyl |
| P | C≡N | 2-thiényl | allyl |
| Q | C≡N | 2-thiényl | 3-triflurométhyl phényl |
| R | NHC₃H₇ | 3-thiényl | méthoxyphényl |
| S | C≡N | 3-thiényl | benzyl |
| T | C≡N | 3-thiényl | méthoxyméthyl |
| U | CO₂Me | 2-thiényl | cyclohexyl |
| V | CO₂Et | 2-thiényl | benzyl |
| W | CO₂Et | 2-thiényl | phenéthyl |
| X | hydroxyméthyle | 3-thiényl | allyl |
| Y | CO₂Me | 3-thiényl | 3,4-dichlorophényl |

## Revendications

1. Procédé de préparation de composés de formule générale I 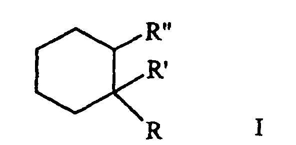 sous forme racémique, de diastéréoisomères ou d'énantiomères, dans laquelle
**R** représente le radical cyano ou -C(O)A dans lequel A représente un radical de formule OR₁ ou NR₂R₃ et R₁, R₂ et R₃ représentent, indépendamment, un atome d'hydrogène ou un groupe alkyl éventuellement substitué par aryl,
**R'** représente le radical 2-thiényl ou 3-thiényl ;
**R"** représente un radical alkyl éventuellement substitué par aryl ; ou un radical aryle, ainsi que les sels de ces produits,
**caractérisé en ce que** l'on fait réagir un produit de formule générale 1
R'CH₂R 1
dans laquelle R et R' ont les significations indiquées ci-dessus,
avec un produit de formule générale 2 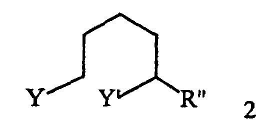 dans laquelle Y et Y' représentent, indépendamment, des groupes partants et R" a la signification indiquée ci-dessus, pour donner un produit de formule I.

2. Procédé selon la revendication 1, pour la préparation de composés de formule générale I sous forme d'énantiomère, **caractérisé en ce que** l'on fait réagir un composé de formule 2 sous forme d'énantiomère.

3. Composés de formule 2 telle définie à la revendication 1, sous forme racémique ou d'énantiomère.

4. Composés de formule générale I : 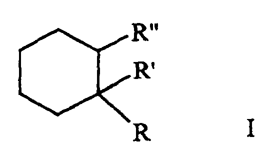 sous forme racémique, de diastéréoisomères ou d'énantiomères, dans laquelle
**R** représente le radical cyano ; un radical de formule -C(O)A dans laquelle A représente un radical de formule OR₁ ou NR₂R₃ dans laquelle R₁, R₂ et R₃ représentent, indépendamment, un atome d'hydrogène ou un groupe alkyl éventuellement substitué par aryl ;
**R'** représente le radical 2-thiényl ou 3-thiényl ;
**R"** représente un radical alkyle éventuellement substitué par aryl ; ou un radical aryle ; ainsi que les sels de ces produits.

5. Composés de formule générale I telle que définie à la revendication 4, dans laquelle
**R** représente le radical cyano ; un radical de formule -C(O)A dans laquelle A représente un radical de formule OR₁ ou NR₂R₃, et R₁, R₂ et R₃ représentent, indépendamment, un atome d'hydrogène, un radical alkyle contenant de 1 à 6 atomes de carbone et éventuellement substitué par phényle ;
**R'** représente le radical 2-thiényl ou 3-thiényl ;
**R"** représente un radical alkyle contenant de 1 à 6 atomes de carbone et éventuellement substitué par phényle ; ou un radical phényle.

6. Composés de formule générale I telle que définie à l'une des revendications 4 à 5, sous forme racémique, de diastéréoisomères ou d'énantiomères, et répondant aux formules suivantes :
- le 2-méthyl-1-(2-thiényl) cyclohexane carbonitrile ;
- l'acide 2-méthyl-1-(2-thiényl) cyclohexane carboxylique ;
- le 2-méthyl-1-(2-thiényl) cyclohexane carboxylate d'éthyle ;
- la 2-méthyl-1-(2-thiényl) cyclohexane carboxamide ;
- la N-[α-méthyl-((S)-phénylméthyl)] 2-méthyl-1-(2-thiényl) cyclohexane carboxamide ;
- le 2-méthyl-1-(3-thiényl) cyclohexane carbonitrile ;
- l'acide 2-méthyl-1-(3-thiényl) cyclohexane carboxylique ;
- la 2-méthyl-1-(3-thiényl) cyclohexane carboxamide ;
- le 2-éthyl-1-(2-thiényl) cyclohexane carbonitrile ;
- le 2-propyl-1-(2-thiényl) cyclohexane carbonitrile ;
- le 2-benzyl-1-(2-thiényl) cyclohexane carbonitrile
- le 2-phényl-1-(2-thiényl) cyclohexane carbonitrile.

## Patentansprüche

1. Verfahren zur Herstellung von Verbindungen der allgemeinen Formel I 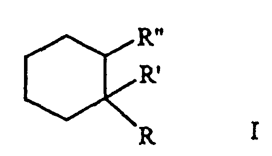 in racemischer Form, in Form von Diastereoisomeren oder von Enantiomeren, in der
**R** den Rest Cyan oder -C(O)A darstellt, worin A einen Rest der Formel OR₁ oder NR₂R₃ darstellt und R₁, R₂ und R₃ unabhängig voneinander ein Wasserstoffatom oder eine ggf. durch Aryl substituierte Alkylgruppe darstellen,
**R'** den Rest 2-Thienyl oder 3-Thienyl darstellt,
**R"** einen ggf. durch Aryl substituierten Alkylrest oder einen Arylrest darstellt,
sowie die Salze dieser Produkte,
**dadurch gekennzeichnet, daß** man ein Produkt der allgemeinen Formel 1
R'CH₂R 1
in der R und R' die oben angegebenen Bedeutungen haben, mit einem Produkt der allgemeinen Formel 2 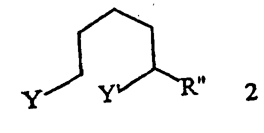 in der Y und Y' unabhängig voneinander Abgangsgruppen darstellen und R" die oben angegebene Bedeutung hat, reagieren läßt, um ein Produkt der Formel I zu erhalten.

2. Verfahren nach Anspruch 1 für die Herstellung von Verbindungen der allgemeinen Formel I in Enantiomerform, **dadurch gekennzeichnet, daß** man eine Verbindung der Formel 2 in Enantiomerform reagieren läßt.

3. Verbindungen der in Anspruch 1 definierten Formel 2 in racemischer Form oder in Enantiomerform.

4. Verbindungen der allgemeinen Formel I 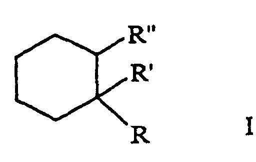 in racemischer Form, in Form von Diastereoisomeren oder von Enantiomeren, in der
**R** den Rest Cyan oder einen Rest der Formel -C(O)A darstellt, in der A einen Rest der Formel OR₁ oder NR₂R₃ darstellt, worin R₁, R₂ und R₃ unabhängig voneinander ein Wasserstoffatom oder eine ggf. durch Aryl substituierte Alkylgruppe darstellen,
**R'** den Rest 2-Thienyl oder 3-Thienyl darstellt,
**R"** einen ggf. durch Aryl substituierten Alkylrest oder einen Arylrest darstellt,
sowie die Salze dieser Produkte.

5. Verbindungen der in Anspruch 4 definierten allgemeinen Formel I, in der
**R** den Rest Cyan oder einen Rest der Formel -C(O)A darstellt, in der A einen Rest der Formel OR₁ oder NR₂R₃ darstellt, worin R₁, R₂ und R₃ unabhängig voneinander ein Wasserstoffatom oder einen ggf. durch Phenyl substituierten Alkylrest mit 1 bis 6 Kohlenstoffatomen darstellen,
**R'** den Rest 2-Thienyl oder 3-Thienyl darstellt,
**R"** einen ggf. durch Phenyl substituierten Alkylrest mit 1 bis 6 Kohlenstoffatomen oder einen Phenylrest darstellt.

6. Verbindungen der in einem der Ansprüche 4 bis 5 definierten allgemeinen Formel I in racemischer Form, in Form von Diastereoisomeren oder von Enantiomeren, die den folgenden Formeln entsprechen:
- 2-Methyl-1-(2-thienyl)cyclohexancarbonitril;
- 2-Methyl-1-(2-thienyl)cyclohexancarbonsäure;
- 2-Methyl-1-(2-thienyl)cyclohexanethylcarboxylat;
- 2-Methyl-1-(2-thienyl)cyclohexancarboxamid;
- N-[α-Methyl-((S)-phenylmethyl)]2-methyl-1-(2-thienyl) cyclohexancarboxamid;
- 2-Methyl-1-(3-thienyl)cyclohexancarbonitril;
- 2-Methyl-1-(3-thienyl)cyclohexancarbonsäure;
- 2-Methyl-1-(3-thienyl)cyclohexancarboxamid;
- 2-Ethyl-1-(2-thienyl)cyclohexancarbonitril;
- 2-Propyl-1-(2-thienyl)cyclohexancarbonitril;
- 2-Benzyl-1-(2-thienyl)cyclohexancarbonitril;
- 2-Phenyl-1-(2-thienyl)cyclohexancarbonitril.

## Claims

1. Process for the preparation of compounds of general formula I 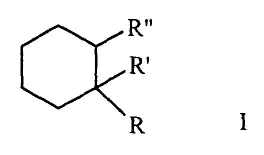 in racemic form, or in the form of diastereoisomers or enantiomers, in which
**R** represents the cyano or -C(O)A radical in which A represents a radical of formula OR₁ or NR₂R₃ and R₁, R₂ and R₃ represent, independently, a hydrogen atom or an alkyl group optionally substituted by aryl ;
**R'** represents the 2-thienyl or 3-thienyl radical;
**R"** represents an alkyl radical optionally substituted by aryl ; or an aryl radical, as well as the salts of these products,
**characterized in that** a product of general formula 1
R'CH₂R 1
in which R and R' have the meanings indicated above,
is reacted with a product of general formula 2 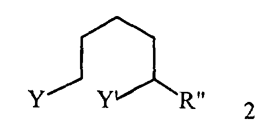 in which Y and Y' represent, independently, parting groups and R" has the meaning indicated above, in order to produce a product of formula I.

2. Process according to claim 1 for the preparation of compounds of general formula I in the form of enantiomer, **characterized in that** a compound of formula 2 in the form of enantiomer is reacted.

3. Compounds of formula 2 as defined in claim 1, in racemic form or in the form of enantiomer.

4. Compounds of general formula I: 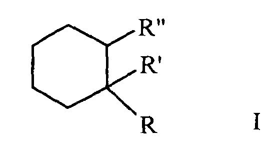 in racemic form, or in the form of diastereoisomers or enantiomers, in which
**R** represents the cyano radical ; a radical of formula -C(O)A in which A represents a radical of formula OR₁ or NR₂R₃ and R₁, R₂ and R₃ represent, independently, a hydrogen atom or an alkyl group optionally substituted by aryl;
**R'** represents the 2-thienyl or 3-thienyl radical;
**R"** represents an alkyl radical optionally substituted by aryl; or an aryl radical ; as well as the salts of these products.

5. Compounds of general formula I as defined in claim 4, in which
**R** represents the cyano radical; a radical of formula -C(O)A in which A represents a radical of formula OR₁ or NR₂R₃ and R₁, R₂ and R₃ represent, independently, a hydrogen atom, an alkyl radical of 1 to 6 carbon atoms optionally substituted by phenyl;
**R'** represents the 2-thienyl or 3-thienyl radical;
**R"** represents an alkyl radical of 1 to 6 carbon atoms optionally substituted by phenyl; or a phenyl radical.

6. Compounds of general formula I as defined in claims 4 to 5, in racemic form, or in the form of diastereoisomers or enantiomers, and corresponding to the following formulae:
- 2-methyl-1-(2-thienyl) cyclohexane carbonitrile;
- 2-methyl-1-(2-thienyl) cyclohexane carboxylic acid;
- ethyl 2-methyl-1-(2-thienyl) cyclohexane carboxylate;
- 2-methyl-1-(2-thienyl) cyclohexane carboxamide;
- N-[α-methyl-((S)-phenylmethyl)] 2-methyl-1-(2-thienyl) cyclohexane carboxamide;
- 2-methyl-1-(3-thienyl) cyclohexane carbonitrile;
- 2-methyl-1-(3-thienyl) cyclohexane carboxylic acid;
- 2-methyl-1-(3-thienyl) cyclohexane carboxamide;
- 2-ethyl-1-(2-thienyl) cyclohexane carbonitrile;
- 2-propyl-1-(2-thienyl) cyclohexane carbonitrile;
- 2-benzyl-1-(2-thienyl) cyclohexane carbonitrile
- 2-phenyl-1-(2-thienyl) cyclohexane carbonitrile.
